# EUROPEAN PATENT SPECIFICATION

(11) **EP 4 151 271 B1**
(45) Date of publication and mention of the grant of the patent: **19.02.2025**
(21) Application number: 21306290.4
(22) Date of filing: 17.09.2021
(51) Int. Cl.: A61N 1/04, A61B 5/11, A61H 1/02, A61N 1/36, A61N 1/372

(54) **METHOD AND SYSTEM TO DETERMINE A PERSONALIZED PROFILE OF STIMULATION CHARGE RATE FOR A SUBJECT USING AN ERGOMETER**
METHODE UND SYSTEM ZUR BESTIMMUNG EINES PERSONALISIERTEN PROFILS EINER STIMULATIONSLADUNGSRATE FÜR EIN SUBJEKT, DAS EIN ERGOMETER VERWENDET
MÉTHODE ET SYSTÈME POUR DÉTERMINER UN PROFIL DE TAUX DE CHARGE DE STIMULATION POUR UN SUJET À L'AIDE D'UN ERGOMÈTRE

(43) Date of publication of application: 22.03.2023
(73) Proprietor: Kurage, 69364 Lyon Cedex 7 (FR)
(72) Inventor: POPOVIC MANESKI, Lana, 11000 Belgrade (RS)
(74) Representative: Icosa

(56) References cited:
- WO-A1-2018/085770
- US-A1- 2007 208 392
- US-A1- 2017 157 396
- US-A1- 2021 228 862
- TONG RAYMOND K Y ET AL: "How to prepare a person with complete spinal cord injury to use surface electrodes for FES trike cycling", 2017 INTERNATIONAL CONFERENCE ON REHABILITATION ROBOTICS (ICORR), IEEE, 17 July 2017 (2017-07-17), pages 801 - 805, XP033141675, DOI: 10.1109/ICORR.2017.8009346
- SARABADANI TAFRESHI AMIREHSAN ET AL: "Modeling the effect of tilting, passive leg exercise, and functional electrical stimulation on the human cardiovascular system", MEDICAL AND BIOLOGICAL ENGINEERING AND COMPUTING, SPRINGER, HEILDELBERG, DE, vol. 55, no. 9, 10 February 2017 (2017-02-10), pages 1693 - 1708, XP036304961, ISSN: 0140-0118, [retrieved on 20170210], DOI: 10.1007/S11517-017-1628-8

## Description

### FIELD OF INVENTION

The present invention relates to the field of ergometers having a functional electrical stimulation (FES) system. In particular, the present invention relates to the field of the calibration of FES systems, that is to say the determination of suitable parameters for providing an electrical stimulation of the muscle(s) of a subject, for the purpose of an exercising program on the ergometer. Especially, the present invention refers to a method for determining a profile of stimulation charge rate for at least one muscle or group of muscles of a subject using the ergometer equipped with a FES system, said profile of stimulation charge rate being specific to the ergometer user.

### BACKGROUND OF INVENTION

Functional Electrical Stimulation (FES) is a well-established rehabilitation technique that delivers pulses of electrical current to the nerves, causing the muscles they innervate to contract. This is possible thanks to the electrodes applied on the skin and linked by cables to a stimulator that generates electrical pulses. These pulses excite the nerve of the weakened or paralyzed muscle so that the muscle contracts.

To date, there are many ergometers (for example, stationary bikes or stationary/indoor rowers or elliptic trainers) equipped with a FES system for the rehabilitation of paralyzed limbs or of limbs requiring rehabilitation. Some of these devices integrate specific functions. For example, the application WO 2018/085770 describes a functional electrical stimulation ergometer incorporating an antispasm control unit, and the application US2021/228862 discloses a functional electrical stimulation system for offering coordinated and natural movements for people with motor system damage, having a computational device with communication unit that allows information exchange with electrical stimulation device.

However, the devices currently available on the market still require the assistance of a qualified person to define the electrical stimulation parameters of the FES system, suitable for its user. This phase, called the device's "calibration phase", is therefore long, tedious and delays the phase of using the ergometer corresponding to the individual's physical training phase.

Moreover, most devices currently available on the market do not allow to calibrate the stimulation charge rate required for the user's limbs that are set in motion by the ergometer. Indeed, in these devices, the user has to configure himself the stimulation parameters among a limited set of accessible parameters and/or pre-recorded sets of parameters.

However, the rate of delivery of the stimulation charge may strongly vary from a user to another depending on the age and/or the motor disability.

There is therefore a need to provide ergometers equipped with a FES system which can automatically calibrate the stimulation pulses parameters (shape, width, current, frequency...) and therefore the charge delivery rate, according to the muscular and/or skeleton specificities of its user and depending on the nature of the exercise and of the periodic movement induced by using the ergometer. Especially, there is a need for providing a finer calibration of the stimulation parameters specific to each user and to each use of the ergometer equipped with the FES system.

### SUMMARY

The invention is defined in claims 1, 9 and 11. Further aspects and preferred embodiments are defined in the appended claims. Aspects, embodiments and examples of the present disclosure which do not fall under the scope of the appended claims do not form part of the invention and are merely provided for illustrative purposes.

### DEFINITION

In the present invention, the following terms have the following meanings:

**"Articulated actuator"** refers to a part of an ergometer for moving at least one user's limb set in motion or engaged in a periodic movement induced by said ergometer. According to one embodiment, the articulated actuator comprises at least one force sensor and/or at least one position sensor, preferably said sensors being located at the joints of the articulated actuator and/or within the contact area between the user and the actuator. According to one embodiment, the articulated actuator is a pedal.

**"Angular position", "angle"** or **"crank angle"** refers, in a cyclic ergometer comprising foot and/or hand pedals such as a stationary bike, to the angle between the crank arm position and the horizontal position, in the crank rotation plane. According to one embodiment, the position of each right or left crank arm defines a crank angle (α; α') to the horizontal position. According to one embodiment, the left pedal and the right pedal of the ergometer are in opposite positions (that-is-to say α = α' +180°).

**"Charge"** or **"electrical charge"** (noted Q) refers to a physical parameter whose unit of measurement is the coulomb (C). A charge of 1 coulomb corresponds to the quantity of electricity carried by an electric current of 1 ampere for 1 second. Thus, the charge may be calculated from the electric current (I, expressed in ampere (A)) and the time (t, expressed in seconds (s)) from equation (e1): Q = I × t (e1). In the present invention, the expression **"stimulation charge rate"** or **"stimulation charge delivery rate"** refers to the charge rate provided by the FES system to the ergometer user. According to one embodiment, the expression **"charge rate"** or **"charge delivery rate"** refers to the charge per pulse multiplied by the pulse frequency, or the amount of the charge delivered to the user per unit of time (in Coulomb per second). According to one embodiment, the expression **"stimulation charge rate personalized to the user's body"** means that the stimulation charge rate is determined by taking into account the physiological condition of the user (muscle strength, muscle spasticity, size of segments for instance) and/or the global geometrical configuration (position of the user or placement of electrodes for instance); preferably at the time of using the ergometer. According to one embodiment, the expression **"stimulation charge rate personalized to the user's body"** means that the stimulation charge rate is determined in an automatic procedure specific to the ergometer user at each start of ergometer use.

**"Dynamic force"** refers to the force induced by at least one muscle or group of muscles of the user's limbs when the user's limbs are set in motion by the ergometer, preferably when the motor of the ergometer is on; said dynamic force being estimated from the force or torque sensors located on the ergometer. According to one embodiment, the **"dynamic force"** is measured at constant stimulation charge rate, preferably as a function of the degree of advancement of the periodic movement (said degree of advancement being expressed from 0% to 100% of the periodic movement). According to one embodiment, the **"passive dynamic force"** is measured when the FES system is off. According to one embodiment, the **"active dynamic force"** is measured when the FES system is on.

**"Ergometer"** refers to a physical exercise machine which consists in making the user reproduce a movement that would make him move if he were not on the machine. According to one embodiment, the ergometer is equipped with an apparatus for measuring the work performed by exercising. According to one embodiment, the ergometer is a cycling ergometer such as a stationary bike or an elliptic trainer. According to one embodiment, the ergometer is equipped with a functional electrical stimulation (FES) system.

**"Exercise regime"** refers to the individual's physical training phase using the ergometer. According to one embodiment, during the exercise regime, the FES system provides an electrical stimulation to the muscle(s) of the user according to the stimulation parameters that have been previously determined in a separate step. According to one embodiment, the exercise regime does not comprise or is not the phase for determining the stimulation parameters (stimulation charge rate and/or stimulation intervals) of a user of the ergometer.

**"Pain threshold"** refers a value of the stimulation charge rate where the user reached the maximum discomfort she or he can sustain. According to one embodiment, the expression "pain threshold" refers to the upper limit of tolerance of the user to pain..

**"Periodic movement"** refers to any movement that recurs, identical to itself. According to one embodiment, the periodic movement is a continuous movement. According to one embodiment, the periodic movement is obtained when the ergometer of the invention sets in motion at least one limb (lower limb and/or upper limb) of a subject using said ergometer. According to one embodiment, the periodic movement is obtained when the ergometer of the invention sets in motion at least one muscle or group of muscles of a lateral part (left and/or right) of a subject using said ergometer. According to one embodiment, at least one muscle or group of muscles of at least one lateral part of the user's body contracts during the periodic movement of said lateral part. According to one embodiment, a **"periodic movement"** corresponds to a movement wherein each single point of the trajectory corresponds to a single value of degree of advancement of the periodic movement (in percentage), for instance a single angle value, or a single value of the crank arm angle. According to embodiment, when the ergometer is a cyclic ergometer comprising foot and/or hand pedals, the degree of advancement of the periodic movement may be defined by the "angle" or "crank angle", used indifferently.

**"Pedal"** refers to each of a pair of foot- and/or hand-operated levers used for turning the main rotation axis of an ergometer.

**"Personalized method"** means, in the present invention, that the method is implemented at each start (or each use) of the ergometer equipped with a FES system so that the determined parameters by the method are recalculated each time the ergometer with the FES system is started. According to one embodiment, the expression "personalized method" means that the determined parameters by the method are not reused from a use session to another.

**"Static force"** refers to the force provided by the contraction of at least one muscle or group of muscles of the user's limbs; said contraction being obtained by the provision to said muscle(s) of a stimulation charge rate by the FES system when the ergometer does not set in motion the user's limbs, preferably when the motor of the ergometer is off; said static force being estimated from the force or torque sensors located on the ergometer. In the present invention, the **"static force profile"** refers to the evolution of the static force as a function of the stimulation charge rate received by the stimulated muscle(s) of the user's body during the implementation of the ramp of the stimulation charge rate.

**"Stimulation charge rate profile"** or **"Stimulation charge delivery rate profile" or "profile of the stimulation charge rate"** refers to the charge interval required for stimulating at least muscle of the user's limbs, said charge interval ranging from a minimum stimulation charge rate (Cₘᵢₙ) required for inducing the contraction onset of said muscle or group of muscles to a maximum stimulation charge rate (Cₘₐₓ) for said muscle or group of muscles corresponding to the charge rate required for achieving the pain threshold of said muscle or group of muscles and/or leading to a capping of the force generated by the contraction of said muscle or group of muscles on the ergometer, preferably on the ergometer pedals. According to one embodiment, the stimulation charge rate profile is specific to each user. According to one embodiment, the stimulation charge rate profile may be deduced from the static force profile.

**"Stimulation charge rate ramp"** or **"Stimulation charge delivery rate ramp"** or **"ramp of stimulation charge rate"** refers to the fact of providing a charge rate by the FES system to a subject, said charge rate varying continuously between a minimum value and a maximum value and being expressed in coulomb per second (C/s), in millicoulomb per second (mC/s) or in nanocoulomb per second (nC/s). According to one embodiment, the ramp of stimulation charge rate ranges from more than 0 to 100 mC/s. According to one embodiment, the ramp of stimulation charge rate does not refer to modulation of the stimulation charge rate in steps.

**"Subject"** or **"user"** refers to a human (male or female). The user may be a child, a teenager, an adult or an elderly person. According to one embodiment, the human has a disability, preferably a motor disability, more preferably is a paraplegic person or a quadriplegic person. According to one embodiment, the human has no sensory ability or a limited sensory ability. According to one embodiment, the human doesn't have any disability. According to one embodiment, the human has a muscle weakness. According to one embodiment, the user's motor disability affects only one lateral part (one side) of the user's body. According to one embodiment, the term **"user"** in the present invention refers to a human that uses the ergometer of the invention.

**"User's limbs"** refers to the upper and/or lower members of a subject; preferably said members being engaged in the movement of the ergometer during its use.

### DETAILED DESCRIPTION

### Method for determining α profile of stimulation charge rate (Method A)

This invention relates to a method for determining a profile of stimulation charge rate for at least one muscle or group of muscles of a subject. According to one embodiment, the method of the invention is a computer-implemented method. According to one embodiment, the method is for calibrating the electrical stimulation parameters, preferably the stimulation charge rate, of a FES system depending on the subject using the FES system and/or an ergometer for training, preferably a cyclic ergometer. According to one embodiment, the ergometer is motorized, preferably said motorized ergometer comprises a motor configured for moving at least on limb of the user's body in a periodic movement. According to one embodiment, the method is for determining at least one profile of stimulation charge rate specific to at least one muscle or group of muscles of a subject. According to one embodiment, the method is a calibration method of a functional electrical stimulation (FES) system, said FES system being integrated or not, to an ergometer; preferably a cyclic ergometer. According to one embodiment, the method of the invention is neither a method of diagnostic of a disease or a disability, nor a method of treatment of the subject. According to one embodiment, the method of the invention allows determining the best conditions of using a FES system that have to be provided later to the user of an ergometer, during an exercise regime. Especially, the method of the invention allows determining the minimum stimulation charge rate (Cₘᵢₙ) and the maximum stimulation charge rate (Cₘₐₓ) for at least one muscle of the body of the user of the ergometer; said minimum stimulation charge rate (Cₘᵢₙ) and maximum stimulation charge rate (Cₘₐₓ) being specific to each user of the ergometer; and preferably specific to each use of said ergometer.

According to one embodiment, the FES system comprises one or more stimulation channels (C). According to one embodiment, the FES system comprises from 1 to 16 stimulation channels. According to one embodiment, the FES system comprises 1, 2, 3, 4, 5, 6, 7, 8, 9, 10, 11, 12, 13, 14, 15 or 16 channels. According to one embodiment, each stimulation channel comprises at least one cathode electrode to be applied on a muscle of a subject. According to one embodiment, the anode electrode is shared for multiple channels. Applying an electrode on a muscle of a subject may be performed for instance by adhering the electrode to the surface of the skin above said muscle

According to one embodiment, the method is implemented in a motorized ergometer, preferably a motorized cyclic ergometer. According to one embodiment, the cyclic ergometer is a stationary bike or an elliptic trainer. According to one embodiment, the (motorized) cyclic ergometer is equipped with a functional electrical stimulation (FES) system. According to one embodiment, the motorized cyclic ergometer comprises a motor configured for moving a left pedal and a right pedal. According to one embodiment, the motorized cyclic ergometer comprises a saddle and a motor configured for moving a left pedal and a right pedal.

According to one embodiment, the invention relates to a method for determining a profile of stimulation charge rate for at least one muscle or group of muscles of a user's body, in a motorized ergometer equipped with a functional electrical stimulation (FES) system; said motorized ergometer comprising a motor configured for moving at least on limb of the user's body in a periodic movement; said stimulation charge rate being provided by the FES system and being personalized to the user's body; said method comprising or consisting of the following steps of:
- Step (a): the placement of the user's limbs in a use position of the motorized ergometer:
- Step (b): the immobilization of the user's limbs in the use position of step (a) by blocking the movement of the motorized ergometer; and
- Step (c): the implementation of a ramp of stimulation charge rate by the FES system for at least one muscle or group of muscles of the user's limbs of step (b) in order to obtain a static force profile for said at least one muscle or group of muscles as a function of the stimulation charge rate of the ramp; said static force being the force applied by the user's limbs on the motorized ergometer; said step being implemented when the user's limbs are immobilized in the position of step (b); and
- Step (d): optionally, the repetition of step (c) for each muscle or group of muscles of the user's limbs for which a stimulation by the FES system has to be provided when using the motorized ergometer.

According to one embodiment, the invention relates to a method for determining a profile of stimulation charge rate for at least one muscle or group of muscles of a user's body, in a motorized cyclic ergometer equipped with a functional electrical stimulation (FES) system; said motorized cyclic ergometer comprising a motor configured for moving a left pedal and a right pedal; said stimulation charge rate being provided by the FES system and being personalized to the user's body; said method comprising or consisting of the following steps of:
- Step (a): the placement of the user's limbs in a use position of the ergometer:
- Step (b): the immobilization of the user's limbs in the use position of step (a) by blocking the right pedal and the left pedal; and
- Step (c): the implementation of a ramp of stimulation charge rate by the FES system for at least one muscle or group of muscles of the user's limbs of step (b) in order to obtain a static force profile for said at least one muscle or group of muscles as a function of the stimulation charge rate of the ramp; said static force being the force applied by the user's limbs on the pedals of the ergometer; said step being implemented when the user's limbs are immobilized in the position of step (b); and
- Step (d): optionally, the repetition of step (c) for each muscle or group of muscles of the user's limbs for which a stimulation by the FES system has to be provided when using the ergometer.

### Steps (a) & (b)

According to one embodiment, the user's limbs may be at least one lower limb and/or upper limb. According to one embodiment, the lower limb may be any part of the leg from the toe until the hip, preferably is selected from the hip, the knee, the ankle, the leg or the foot. According to one embodiment, the upper limb may be any part of the arm from the toe until the shoulder, preferably is selected from the arm (the upper arm), the forearm (the lower arm), and the hand.

In the present invention, a "use position" in step (a) refers to a position in which the user is installed on the ergometer and in which the user is ready to use it. According to one embodiment, in a motorized cyclic ergometer comprising a saddle and a crankset, a use position may be the position in which the user seats on the saddle and places his left foot on the left pedal and the right foot on the right pedal of said ergometer; preferably the line that crosses the left and right pedals is perpendicular to the line that goes from the user's pelvis to the hub of the crankset, more preferably the crankset is further blocked in this position. According to one embodiment, "a use position" refers to a position of the user on a motorized cyclic ergometer in which the user's limbs are placed on the crankset of said ergometer which is blocked so that to avoid any unintentional movement of the user's limbs on said ergometer. According to one embodiment, "a use position" refers to a position of the user ready to use the ergometer, preferably the motorized ergometer; preferably in which the user's limbs engaged in the periodic movement are blocked so as to avoid any unintentional movement of the user's limbs on said ergometer.

### Step (c)/ step (d)

According to one embodiment, the stimulation charge rate of the ramp in step (c) ranges from more than 0 mC/s to 100 mC/s. According to one embodiment, the stimulation charge rate of the ramp ranges from more than 0 µC/s to 50 µC/s; preferably from 0.1 µC/s to 20 µC/s. According to one embodiment, Cₘᵢₙ or Cₘₐₓ is 1, 2, 3, 4, 5, 6, 7, 8, 9, 10, 11, 12, 13, 14, 15, 16, 17, 18, 19 or 20 µC/s. According to one embodiment, the stimulation charge rate of the ramp ranges from 5 µC/s to 50 µC/s, 10 µC/s to 50 µC/s, 15 µC/s to 50 µC/s, 20 µC/s to 50 µC/s, 25 µC/s to 50 µC/s, 30 µC/s to 50 µC/s, 35 µC/s to 50 µC/s, 40 µC/s to 50 µC/s, or `5 µC/s to 50 µC/s. According to one embodiment, the stimulation charge rate of the ramp ranges from more than 0 µC/s to 50 µC/s, from more than 0 µC/s to 45 µC/s, from more than 0 µC/s to 40 µC/s, from more than 0 µC/s to 35 µC/s, from more than 0 µC/s to 30 µC/s, from more than 0 µC/s to 25 µC/s, from more than 0 µC/s to 20 µC/s, from more than 0 µC/s to 15 µC/s, from more than 0 µC/s to 10 µC/s, or from more than 0 µC/s to 5 µC/s. According to one embodiment, the stimulation charge rate of the ramp 0.1, 0.2, 0.3, 0.4, 0.5, 0.6, 0.7, 0.8, 0.9 or 1 µC/s. According to one embodiment, the stimulation charge rate of the ramp ranges from 0.05 mC/s to 100 mC/s, from 0.5 mC/s to 100 mC/s, from 1 mC/s to 100 mC/s, from 5 mC/s to 100 mC/s, from 10 mC/s to 100 mC/s, from 20 mC/s to 100 mC/s, from 30 mC/s to 100 mC/s, from 40 mC/s to 100 mC/s, from 50 mC/s to 100 mC/s, from 60 mC/s to 100 mC/s, from 70 mC/s to 100 mC/s, from 80 mC/s to 100 mC/s, or from 90 mC/s to 100 mC/s.

According to one embodiment, the ramp of stimulation charge rate is a ramp of stimulation electric current. According to one embodiment, the stimulation electric current ramp ranges from more than 0 mA to 200 mA, preferably ranges from 50 mA to 150 mA, more preferably is 100 mA. According to one embodiment, the stimulation electric current ramp ranges from 0.1 mA to 200 mA, from 1 mA to 200 mA, from 10 mA to 200 mA, from 20 mA to 200 mA, from 30 mA to 200 mA, from 40 mA to 200 mA, from 50 mA to 200 mA, from 60 mA to 200 mA, from 70 mA to 200 mA, from 80 mA to 200 mA, from 90 mA to 200 mA, from 100 mA to 200 mA, from 110 mA to 200 mA, from 120 mA to 200 mA, from 130 mA to 200 mA, from 140 mA to 200 mA, from 150 mA to 200 mA, from 160 mA to 200 mA, from 170 mA to 200 mA, from 180 mA to 200 mA or from 190 mA to 200 mA. According to one embodiment, the stimulation electric current ramp ranges from 0.1 mA to 190 mA, from 0.1 mA to 180 mA, from 0.1 mA to 170 mA, from 0.1 mA to 160 mA, from 0.1 mA to 150 mA, from 0.1 mA to 140 mA, from 0.1 mA to 130 mA, from 0.1 mA to 120 mA, from 0.1 mA to 110 mA, from 0.1 mA to 100 mA, from 0.1 mA to 90 mA, from 0.1 mA to 80 mA, from 0.1 mA to 70 mA, from 0.1 mA to 60 mA, from 0.1 mA to 50 mA, from 0.1 mA to 40 mA, from 0.1 mA to 30 mA, from 0.1 mA to 20 mA, or from 0.1 mA to 10 mA. According to one embodiment, the stimulation electric current ramp is implemented with a fixed value of pulse width; preferably said fixed value of pulse width is selected in a range from more than 0 to 1000 µs, preferably is 350 µs.

According to one embodiment, the ramp of stimulation charge rate is a stimulation pulse width ramp. According to one embodiment, the stimulation pulse width ramp ranges from more than 0 to 1000 µs, preferably from 0.1 µs to 500 µs, more preferably is 350 µs. According to one embodiment, the stimulation pulse width ramp ranges from 0.1 to 1000 µs, from 1 to 1000 µs, from 10 to 1000 µs, from 100 to 1000 µs, from 200 to 1000 µs, from 300 to 1000 µs, from 400 to 1000 µs, from 500 to 1000 µs, from 600 to 1000 µs, from 700 to 1000 µs, from 800 to 1000 µs, or from 900 to 1000 µs. According to one embodiment, the stimulation pulse width ramp ranges from 0.1 to 900 µs, from 0.1 to 800 µs, from 0.1 to 700 µs, from 0.1 to 600 µs, from 0.1 to 500 µs, from 0.1 to 400 µs, from 0.1 to 300 µs, from 0.1 to 200 µs, from 0.1 to 100 µs or from 0.1 to 10 µs. According to one embodiment, the stimulation pulse width ramp is implemented with a fixed value of electric current; preferably said fixed value of electric current is selected in a range from more than 0 to 200 mA, preferably is 100 mA.

According to one embodiment, the ramp of stimulation charge rate is a combination of a stimulation electric current ramp and a stimulation pulse width ramp. According to one embodiment, the stimulation pulse width ramp ranges from more than 0 to 1000 µs and the stimulation electric current ramp ranges from more than 0 mA to 200 mA

According to one embodiment, the ramp of stimulation charge rate may be implemented with a frequency ranging from more than 0 to 100 Hz, preferably from 0.1 Hz to 50 Hz, more preferably is 40 Hz. According to one embodiment, the frequency ranges from 0.1 Hz to 100 Hz, from 1 Hz to 100 Hz, from 10 Hz to 100 Hz, from 20 Hz to 100 Hz, from 30 Hz to 100 Hz, from 40 Hz to 100 Hz, from 50 Hz to 100 Hz, from 60 Hz to 100 Hz, from 70 Hz to 100 Hz, from 80 Hz to 100 Hz, or from 90 Hz to 100 Hz. According to one embodiment, the frequency ranges from 0.1 Hz to 90 Hz, from 0.1 Hz to 80 Hz, from 0.1 Hz to 70 Hz, from 0.1 Hz to 60 Hz, from 0.1 Hz to 50 Hz, from 0.1 Hz to 40 Hz, from 0.1 Hz to 30 Hz, from 0.1 Hz to 20 Hz, or from 0.1 Hz to 10 Hz.

According to one embodiment, the time of the ramp of stimulation charge rate in step (c) ranges from 1s to 3600s; preferably from 1s to 60s; more preferably is 10s. According to one embodiment, the time of the stimulation charge rate ramp in step (c) is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 s.

According to one embodiment, the user's limbs are the user's legs. According to one embodiment, step (c) is simultaneously implemented for the same kind of muscle or group of muscles of both user's legs.

According to one embodiment, the muscle or group of muscles of the user's body is selected from: quadriceps (vastus lateralis), quadriceps (vastus medialis), quadriceps (rectus femoris), hamstrings, gluteus, gastrocnemius and tibialis anterior.

According to one embodiment, the ramp of stimulation charge rate by the FES system for at least one muscle or group of muscles of the user's limbs, is implemented by placing at least one electrode of the FES system on at least one muscle of the user's limb for which a stimulation will be provided.

According to one embodiment, the ramp of stimulation charge rate ends when a static force plateau and/or when the pain threshold for the muscle or group of muscles of the user's body receiving the stimulation charge rate, is obtained.

According to one embodiment, when the user is a paralyzed person or a person having a degraded or absent motor ability, or a degraded or absent sensory ability of at least one limb, the stimulation charge ramp of stem (c) ends when a static force plateau for the muscle or group of muscles of the user's body receiving the stimulation charge rate, is obtained. According to one embodiment, the paralyzed person or a person having a degraded or no voluntary motor ability of at least one limb, is a paraplegic or a quadriplegic person.

According to one embodiment, when the user is a valid person, the ramp of stimulation charge rate of step (c) ends when the pain threshold for the muscle or group of muscles of the user's body receiving the stimulation charge rate, is obtained. In the present invention, the expression **"valid person"** refers to a subject having no motor disability.

### Step (e)

According to one embodiment, the method for determining a profile of stimulation charge rate for at least one muscle or group of muscles of a user's body, further comprises a step (e) for determining both the minimum stimulation charge rate (Cₘᵢₙ) required for inducing the contraction onset of said muscle or group of muscles; and the maximum stimulation charge rate (Cₘₐₓ) for said muscle or group of muscles corresponding to the charge required for achieving the pain threshold of said muscle or group of muscles and/or leading to a capping of the force generated by the contraction of said muscle or group of muscles on the ergometer.

### Method for determining stimulation intervals of a periodic movement (Method B)

This disclosure further relates to a method for determining stimulation intervals of a periodic movement for at least one muscle or group of muscles of a subject using an ergometer, preferably using a cyclic ergometer, equipped with a FES system. In the present invention, the expression **"stimulation intervals"** refers to the sections of the periodic movement induced by the ergometer, during which the FES system provides an electrical stimulation to the targeted muscle(s) of the user's limbs set in motion by the ergometer.

According to one embodiment, the method B of the invention is a computer-implemented method. According to one embodiment, the method B is for determining at least one stimulation interval specific to at least one muscle or group of muscles of a subject. According to one embodiment, the method B is a calibration method of a functional electrical stimulation (FES) system, said FES system being integrated or not, to an ergometer. According to one embodiment, the method B of the invention is neither a method of diagnostic of a disease or a disability, nor a method of treatment of the subject. According to one embodiment, the method B of the invention allows determining the best conditions of using a FES system that have to be provided later to the user of an ergometer, during an exercise regime. According to one embodiment, the method B of the invention is for determining the stimulation intervals to be delivered to one or more muscles of the user's body in order to reduce muscle fatigue and/or improve the performances of the user when training on the ergometer in an exercise regime.

According to one embodiment, the method B of the invention comprises or consists of the following steps:
i. recording the passive and active dynamic force profiles for at least one muscle or group of muscles of the user's body, said muscle(s) of the user's body being set in motion or engaged in a periodic movement by the ergometer; and
ii. determining the stimulation intervals for each muscle or group of muscles of the user's body, set in motion or engaged in a periodic movement by the ergometer; as a function of the degree of advancement in the periodic movement (said degree of advancement being expressed in percentages (%)).

### Step (i)

According to one embodiment, step (i) is implemented by recording the dynamic force applied by the user on the articulated actuator or the ergometer when the FES system is on (passive dynamic force, i.e. when the FES system provides a stimulation to any muscle or group of muscles of the user's body) and when the FES system is off (active dynamic force, i.e. when the FES system does not provide any stimulation to any muscle or group of muscles of the user's body); said dynamic force being measured as a function of the degree of advancement in the periodic movement (expressed in percentage).

According to one embodiment, the dynamic force is recorded at step (i) by at least one force sensor or torque sensor located on the articulated actuator or the ergometer, preferably located at its joints.

According to one embodiment, the degree of advancement in the periodic movement ranges from 0% (no advancement in the periodic movement) to 100% (corresponding to one periodic movement). According to one embodiment, the degree of advancement in the periodic movement is 5, 10, 15, 20, 25, 30, 35, 40, 45, 50, 55, 60, 65, 70, 75, 80, 85, 90, 95 or 100%.

According to one embodiment, when the ergometer is a cycle ergometer comprising foot and/or hand pedals such as a stationary bike, the ergometer sets in motion the arms and/or the legs of the user.

According to one embodiment, when the ergometer is a cycle ergometer comprising foot and/or hand pedals such as a stationary bike, the degree of advancement of the periodic movement may be expressed as a function of the angle between the crank arm position and the horizontal position, in the crank rotation plane; said angle is called **"angular position"** or **"angle"** or **"crank angle".** According to one embodiment, the left pedal and the right pedal of the ergometer are in opposite positions (that-is-to say α = α' +180°). According to one embodiment, the angle, or angular position, ranges from 0° to 360°.

According to one embodiment, step (i) is implemented at a constant charge rate value, preferably ranging from more than 0 C/s to 100 mC/s.

According to one embodiment, step (i) is repeated as many times as there are muscles to stimulate by the FES system during the exercise regime.

According to one embodiment, step (i) is implemented during a number of movement phases, preferably during a number of periodic movement phases, ranging from more than 0 to 10; preferably from 1 to 5; more preferably from 1 to 2. According to one embodiment, the passive and/or active dynamic force profiles in step (i) is(are) recorded during a number of periodic movement phases equal to 1 or 2.

### Step (ii)

According to one embodiment, step (ii) is implemented by an algorithm of the ergometer or the FES system.

According to one embodiment, step (ii) comprises or consists of determining the differential dynamic force profile from the passive and active dynamic force profiles recorded at step (i); said differential dynamic force profile as a function of the degree of advancement of the periodic movement.

According to one embodiment, the differential dynamic force profile is specific to each user, each use of the ergometer and/or the nature of the training exercise.

According to one embodiment, the training exercise may be an endurance exercise (that-is-to say an exercise in which the targeted muscle of the user has to maintain a physical effort of low physical constraint on a long time period) or a muscle power exercise (that-is-to say an exercise in which the targeted muscle of the user has to maintain a physical effort with a high physical constraint on a short time period). In the present invention, the expression **"stimulated muscle"** or **"targeted muscle"** means that said muscle received electrical pulses by the FES system of the ergometer.

### Method for calibrating the use parameters of a FES system associated with an ergometer (Method C)

The present disclosure also relates to a method for calibrating the use parameters of a FES system associated with an ergometer (Method C). According to one embodiment, the method C of the invention is specific to each user and/or each use of the ergometer.

According to one embodiment, the method for calibrating the use parameters of a FES system associated with an ergometer (method C) comprises or consists of the following steps:
(c 1) Determining a profile of stimulation charge rate for at least one muscle or group of muscles of the user's body according to the method A as described above; and
(c2) Determining stimulation intervals for at least one muscle or group of muscles of the user's body according to the method B as described above.

According to one embodiment, the method C of the invention is implemented before using the ergometer in an exercise regime. According to one embodiment, in the method C, step (c1) is implemented before implementing step (c2).

According to one embodiment, the method C further comprises a step for selecting the nature of the exercise regime (c1 bis), preferably implemented after (c1), more preferably implemented after (c1) and before (c2).

According to one embodiment, step (c2) is implemented at a constant stimulation charge rate selected in the stimulation charge rate profile obtained at step (c1). According to one embodiment, step (c2) is implemented at a constant stimulation charge rate selected in the range from Cₘᵢₙ to Cₘₐₓ as defined in the method A above. According to one embodiment, the constant stimulation charge rate of step (c2) is selected depending on the nature of the exercise regime. According to one embodiment, the constant stimulation charge rate of step (c2) is selected in the range from Cₘᵢₙ to Cₘₐₓ/2 when the exercise regime is an endurance exercise. According to one embodiment, the constant stimulation charge rate of step (c2) is selected in the range from Cₘₐₓ/2 to Cₘₐₓ when the exercise regime is a muscle power exercise.

### Computer program

The present invention also refers to a computer program comprising instructions to operate the method for determining a profile of stimulation charge rate as defined above (method A) and/or the method for stimulation intervals for a periodic movement (method B) as defined above.

According to one embodiment, the computer program is integrated in the ergometer and/or in the FES system.

### FES system

The present invention also refers to a FES system comprising instructions to operate the method for determining a profile of stimulation charge rate as defined above (method A) and/or the method for stimulation intervals for a periodic movement (method B) as defined above.

According to one embodiment, the FES system of the invention comprises or consists of:
- a module, preferably a computer-based module, comprising a data acquisition system and a FES controller; and
- a stimulator connected to one or more electrical stimulation electrodes; said electrical stimulation electrodes being configured for (i) adhering to at least one targeted muscle or group of muscles of a user, set in motion by the ergometer, and (ii) providing an interface to transmit electrical stimulation signals from the stimulator in electrical impulses to the targeted muscle or group of muscles of the user.

According to one embodiment, the FES system of the invention comprises a storage unit for storing data recorded at step (c) and/or step (e) of the method A and/or recorded at step (i) of the method B.

According to one embodiment, the FES system of the invention comprises a computer program as defined above.

According to one embodiment, the module or the computer-based module of the FES system of the invention may implement the methods A and/or B of the invention as defined above. According to one embodiment, the module or the computer-based module of the FES system of the invention may implement the method C of the invention as defined above.

### Ergometer

The present invention also refers to an ergometer, preferably a cyclic ergometer, equipped with a FES system According to one embodiment, the FES system is as defined above. According to one embodiment, the ergometer is a cyclic ergometer, preferably a stationary bike (also called an electrical stimulation stationary bike). According to one embodiment, the ergometer comprises at least one articulated actuator, preferably comprising at least one position and/or force sensor. According to one embodiment, the position and/or force sensor is located at the joints of articulated actuator.

According to one embodiment, when the ergometer is a stationary bike, the articulated actuator is a foot and/or hand-pedal. According to one embodiment, when the ergometer is a stationary bike, the ergometer comprises at least two foot and/or hand-pedals: one left pedal and one right pedal.

According to one embodiment, when the ergometer is an indoor rower, the articulated actuator is the saddle of the indoor rower.

According to one embodiment, the ergometer of the invention comprises means for implementing the method A and /or the method B of the invention as defined above. According to one embodiment, the ergometer of the invention comprises a FES system comprising instructions for implementing the method A and /or the method B of the invention as defined above.

According to one embodiment, the ergometer of the invention comprises a module able to implement any steps (c), (d) and/or (e) of the method A as defined above. According to one embodiment, the ergometer of the invention comprises a module able to implement any steps (i) and/or (ii) of the method B as defined above.

According to one embodiment, the ergometer of the invention comprises or consists of:
- means for setting in motion or engaging in a periodic movement at least one muscle or group of muscle of the user's body; and
- a FES system.

According to one embodiment, the ergometer comprises means for setting in motion or engaging in a periodic movement at least one muscle or group of muscle of the user's body. According to one embodiment, means for setting in motion or engaging in a periodic movement at least one muscle or group of muscle of the user's body comprise or consist of at least one articulated actuator; the position of the articulated actuator being determined as a function of the degree of advancement in the periodic movement (expressed in percentage).

According to one embodiment, when the ergometer is a cycling ergometer, the ergometer comprises a pair of articulated actuators, preferably a right articulated actuator for the right lateral part of the user's body and a left articulated actuator for the left lateral part of the user's body; the position of each articulated actuator being determined as a function of the degree of advancement in the periodic movement (expressed in percentage).

According to one embodiment, when the ergometer is a cyclic ergometer with a cycle motor, means for setting in motion or engaging in a periodic movement at least one muscle or group of muscle of the user's body comprise or consist of: a pair of pedals consisting of a right pedal and a left pedal; each pedal being connected to the motor by a crank arm; the position of each left and right crank arm defining a crank angle (α; α') to the horizontal position; the right pedal or right crank arm comprising at least one sensor configured for determining the force applied on said right pedal or right crank arm as a function of the crank angle (α) of the right crank arm; and the left pedal or left crank arm comprising at least one sensor configured for determining the force applied on said left pedal or left crank arm as a function of the crank angle (α') of the left crank arm. According to one embodiment, when the ergometer is a cyclic ergometer, the periodic movement is a periodic circular movement.

### Uses

The present invention also refers to the use of the method A as defined above. The present invention also refers to the use of the method C of the invention as defined above. The present invention also refers to the use of a computer program and/or a FES system as defined above.

According to one embodiment, the method A, the method C, the computer program and/or the FES system of the invention is(are) useful for providing one or more electrical pulses to at least one muscle of the user of the ergometer.

According to one embodiment, the method A, the method C, the computer program and/or the FES system of the invention is(are) useful for reducing the muscle fatigue when training on an ergometer.

According to one embodiment, the method A, the method C, the computer program and/or the FES system of the invention is(are) useful for adapting the stimulation charge rate to each user depending on his physiological conditions and/or depending on the nature of the exercise regime.

According to one embodiment, the exercise regime may be an endurance exercise or a muscle power exercise.

According to one embodiment, the method A, the method C, the computer program and/or the FES system of the invention is(are) useful for personalizing the calibration process of the use parameters of the ergometer equipped with a FES system.

According to one embodiment, the method A, the method C, the computer program and/or the FES system of the invention is(are) useful for providing a maximum mechanical efficiency to the user of the ergometer.

According to one embodiment, the method A, the method C, the computer program and/or the FES system of the invention is(are) useful for the increase of muscle mass and strength of a disabled or valid person.

According to one embodiment, the method A, the method C, the computer program and/or the FES system of the invention is(are) useful for the rehabilitation of movement of a disabled person.

### EXAMPLES

The present invention is further illustrated by the following example.

### Example 1: Method for calibrating the use parameters of a FES system associated with an ergometer

In the present invention, the ergometer is a stationary bike equipped with a FES system.

### Step 1: Method for determining the profile of stimulation charge rate of an ergometer user (method A)

First, the user of the ergometer seats on the saddle of the stationary bike.

Then, the user places his left foot on the left pedal and the right foot on the right pedal of the ergometer. The crankset of the ergometer is blocked in a predefined position in which the line crossing the two pedals is perpendicular to the line that goes from the user's pelvis to the hub of the crankset. This position is maintained during the whole process for determining the stimulation charge.

In a next step, at least one electrode of the FES system is placed on the muscle or group of muscles for which the stimulation charge rate has to be determined. A ramp of stimulation charge rate is then carried out by sending an electrical current so that the charge continuously varies from more than 0 C/s up to achieve either the pain threshold of said muscle(s) or the capping of the muscular static force of the user's limbs on the pedals of the ergometer; said static force being measured by at least one force sensor located on each pedal of the crankset. This step is repeated as many times as there are targeted muscles to be stimulated.

For each stimulated muscle, the static force profile is achieved as a function of the stimulation charge rate of the ramp. In this way, for each stimulated muscle of the user's body, a profile of stimulation charge rate is obtained corresponding to the range from a minimum stimulation charge rate (Cₘᵢₙ) required for inducing the contraction onset of said muscle or group of muscles; and the maximum stimulation charge rate (Cₘₐₓ) for said muscle or group of muscles corresponding to the charge rate required for achieving the pain threshold of said muscle or group of muscles and/or leading to a capping of the force generated by the contraction of said muscle or group of muscles on the ergometer pedals.

Before each use of the ergometer, said procedure is implemented in order to determine the profile of stimulation charge rate specific to the physical condition of the user at the time of using the ergometer.

### Step 2: Method for determining stimulation intervals for at least one muscle or group of muscles of a subject using the stationary bike equipped with a FES system (method B)

In a second step, the motor of the ergometer is turned on in order to set in motion the legs of the user in a periodic movement. The FES system is first turned off and the passive dynamic force of the legs applied on the ergometer pedals are recorded as a function of the degree of the advancement of the user's limbs in the periodic movement.

Then, the FES system is turned on and the stimulation charge rate is selected depending on the nature of the exercise chosen and the profile of stimulation charge rate of the user determined in the first step. If the exercise is an endurance exercise, the charge rate is fixed at a constant value selected in the range from Cₘᵢₙ to Cₘₐₓ/2. If the exercise is a muscle power exercise, the charge rate is fixed at a constant value chosen in the range from Cₘₐₓ/2 to Cₘₐₓ.

The active dynamic force for each stimulated muscle or group of muscles of the user's legs are recorded as a function of the degree of advancement of the user's limbs in the periodic movement.

After that, the algorithm of the FES system provides for each stimulated muscle or group of muscles of the user, the differential dynamic force profile as a function of the degree of advancement of the user's limbs in the periodic movement.

In this way, stimulation intervals as a function of the degree of advancement of the users' limbs in the periodic movement, are defined.

Advantageously, the profile of stimulation charge rate obtained from the method A and the stimulation intervals obtained from the method B are specific to each user and to each use of the ergometer so that this self-calibration process provides in a fast and easy procedure, evolutive stimulation parameters for the user and consequently, a more personalized training.

## Claims

1. A calibration method for determining a profile of stimulation charge rate for at least one muscle or group of muscles of a user's body, in a motorized ergometer equipped with a functional electrical stimulation (FES) system; said motorized ergometer comprising a motor configured for moving at least one articulated actuator; said stimulation charge being provided by the FES system and being personalized to the user's body; said method comprising the following steps of:
- Step (a): the placement of the user's limbs in a use position of the ergometer:
- Step (b): the immobilization of the user's limbs in the use position of step (a) by blocking the at least one articulated actuator; and
- Step (c): the implementation of a ramp of stimulation charge rate by the FES system for at least one muscle or group of muscles of the user's limbs of step (b) in order to obtain a static force profile for said at least one muscle or group of muscles as a function of the stimulation charge rate of the ramp; said static force being the force applied by the user's limbs on a part of the ergometer comprising at least one force sensor; said step being implemented when the user's limbs are immobilized in the position of step (b); and
- Step (d): optionally, the repetition of step (c) for each muscle or group of muscles of the user's limbs for which a stimulation by the FES system has to be provided when using the ergometer.

2. The calibration method according to claim **1,** further comprising a step (e) for determining both the minimum stimulation charge rate (Cₘᵢₙ) required for inducing the contraction onset of said muscle or group of muscles; and the maximum stimulation charge rate (Cₘₐₓ) for said muscle or group of muscles corresponding to the charge required for achieving the pain threshold of said muscle or group of muscles and/or leading to a capping of the force generated by the contraction of said muscle or group of muscles on the ergometer.

3. The calibration method according to claim **1** or claim **2,** wherein the user's limbs are the user's legs, and wherein step (c) is simultaneously implemented for the same kind of muscle or group of muscles of both user's legs.

4. The calibration method according to any one of claims **1** to **3,** wherein, in the step (c), the ramp of stimulation charge rate ends when a static force plateau and/or when the pain threshold for the muscle or group of muscles of the user's body receiving the stimulation charge rate, is obtained.

5. The calibration method according to any one of claims **1** to **4,** wherein the time of the ramp of stimulation charge rate ranges from 1s to 60s, preferably is 10s.

6. The calibration method according to any one of claims **1** to **5,** wherein the stimulation charge rate ranges from more than 0 mC/s to 100 mC/s.

7. The calibration method according to any one of claims **1** to **6,** wherein when the user is a paralyzed person or a person having a degraded or absent motor ability, a degraded or absent sensory ability of at least one limb, the ramp of stimulation charge rate of step (c) ends when a static force plateau for the muscle or group of muscles of the user's body receiving the stimulation charge rate, is obtained.

8. The calibration method according to any one of claims **1** to **6,** wherein when the user is a valid person, the ramp of stimulation charge rate of step (c) ends when the pain threshold for the muscle or group of muscles of the user's body receiving the stimulation charge rate, is obtained.

9. A computer program in a motorized ergometer, preferably in a motorized cycling ergometer, equipped with a FES system, said computer program comprising instructions which, when the program is executed by a computer, cause the computer to carry out the steps of the calibration method according to any one of claims **1** to **8.**

10. The computer program according to claim **9,** further comprising a program executed by the computer causing the computer to carry out a method for determining the stimulation intervals for a periodic movement for at least one muscle or group of muscles of the user's body as function of the degree of advancement of the periodic movement on the ergometer; said method being implemented after the implementation of the steps of the calibration method according to any one of claims **1** to **8.**

11. An electric stimulation motorized ergometer comprising:
- a motor configured for moving in a periodic movement at least one articulated actuator comprising at least one position sensor, said articulated actuator being blockable in a use position so as to immobilize the user's limb;
- a functional electrical stimulation (FES) system comprising:
- a module, preferably a computer-based module, comprising a data acquisition system and a FES controller; and
- a stimulator connected to two or more electrical stimulation electrodes; said electrical stimulation electrodes being configured for adhering to at least one muscle or group of muscles of a user's body being engaged in a periodic movement by the motorized ergometer, and for providing an interface to transmit electrical pulses from the stimulator to said muscle or group of muscles of the user's body;
wherein:
said FES controller comprising instructions to operate the calibration method for determining a profile of stimulation charge rate of at least one muscle or group of muscles of a user's body according to any one of claims **1** to **8.**

12. The electric stimulation motorized ergometer according to claim **11,** wherein said motorized ergometer is configured for implementing the calibration method of any one of claims **1** to **8,** before implementation of an exercise regime by a user.

13. The electric stimulation motorized ergometer according to claim **11** or claim **12,** wherein the muscle or group of muscles of the user's body is selected from: quadriceps (vastus lateralis), quadriceps (vastus medialis), quadriceps (rectus femoris), hamstrings, gluteus, gastrocnemius and tibialis anterior.

14. The electric stimulation motorized ergometer according to any one of claims **11** to **13,** wherein the FES system comprises a user interface for:
- implementing the calibration method according to any one of claims **1** to **8,**
- optionally, in a second step, implementing a method for determining the stimulation intervals for a periodic movement for at least one muscle or group of muscles of the user's body as function of the degree of advancement of the periodic movement on the ergometer and as function of the kind of the exercise regime; and
- optionally, in a last step, implementing an exercise regime.

15. The electric stimulation motorized ergometer according to claim**14,** wherein the stimulation charge rate provided by the FES system during the calibration method for determining stimulation intervals of a periodic movement for at least one muscle or group of muscles of the user's body or during the exercise regime ranges from the minimum stimulation charge rate (Cₘᵢₙ) to the maximum stimulation charge rate (Cₘₐₓ) determined for each muscle or group of muscles of each user of the ergometer during the step (e) of the method of claim **2.**

## Patentansprüche

1. Kalibrierungsverfahren zum Bestimmen eines Profils der Stimulationsladungsrate für mindestens einen Muskel oder eine Muskelgruppe des Körpers eines Benutzers in einem motorisierten Ergometer, das mit einem funktionellen elektrischen Stimulationssystem (FES-System) ausgestattet ist; wobei das motorisierte Ergometer einen Motor umfasst, der dazu konfiguriert ist, mindestens ein Gelenkstellglied zu bewegen; wobei die Stimulationsladung vom FES-System bereitgestellt wird und an den Körper des Benutzers individuell angepasst ist; wobei das Verfahren die folgenden Schritte umfasst:
- Schritt (a): das Platzieren der Gliedmaßen des Benutzers in einer Gebrauchsposition des Ergometers;
- Schritt (b): das Ruhigstellen der Gliedmaßen des Benutzers in der Gebrauchsposition von Schritt (a) durch Blockieren des mindestens einen Gelenkstellglieds; und
- Schritt (c): das Durchführen eines Anstiegs der Stimulationsladungsrate durch das FES-System für mindestens einen Muskel oder eine Muskelgruppe der Gliedmaßen des Benutzers von Schritt (b), um ein statisches Kraftprofil für den mindestens einen Muskel oder die eine Muskelgruppe in Abhängigkeit von der Stimulationsladungsrate des Anstiegs zu erhalten; wobei die statische Kraft die Kraft ist, die von den Gliedmaßen des Benutzers auf einen Teil des Ergometers, das mindestens einen Kraftsensor umfasst, ausgeübt wird; wobei der Schritt durchgeführt wird, wenn die Gliedmaßen des Benutzers in der Position von Schritt (b) ruhiggestellt sind; und
- Schritt (d): gegebenenfalls das Wiederholen von Schritt (c) für jeden Muskel oder jede Muskelgruppe der Gliedmaßen des Benutzers, für die bei der Nutzung des Ergometers eine Stimulation durch das FES-System bereitgestellt werden muss.

2. Kalibrierungsverfahren nach Anspruch 1, ferner umfassend einen Schritt (e) zum Bestimmen sowohl der minimalen Stimulationsladungsrate (Cₘᵢₙ), die zum Auslösen des Kontraktionsbeginns des Muskels oder der Muskelgruppe erforderlich ist, als auch der maximalen Stimulationsladungsrate (Cₘₐₓ) für den Muskel oder die Muskelgruppe, die der Ladung entspricht, die zum Erreichen der Schmerzschwelle des Muskels oder der Muskelgruppe erforderlich ist und/oder die zu einer Begrenzung der durch die Kontraktion des Muskels oder der Muskelgruppe auf dem Ergometer erzeugten Kraft führt.

3. Kalibrierungsverfahren nach Anspruch 1 oder Anspruch 2, wobei die Gliedmaßen des Benutzers die Beine des Benutzers sind, und wobei Schritt (c) gleichzeitig für dieselbe Art von Muskel oder Muskelgruppe beider Beine des Benutzers durchgeführt wird.

4. Kalibrierungsverfahren nach einem der Ansprüche 1 bis 3, wobei im Schritt (c) der Anstieg der Stimulationsladungsrate endet, wenn eine statische Kraftebene und/oder wenn die Schmerzschwelle für den Muskel oder die Muskelgruppe des Körpers des Benutzers, der bzw. die die Stimulationsladungsrate empfängt, erreicht wird.

5. Kalibrierungsverfahren nach einem der Ansprüche 1 bis 4, wobei die Zeit des Anstiegs der Stimulationsladungsrate im Bereich von 1 s bis 60 s liegt und vorzugsweise 10 s beträgt.

6. Kalibrierungsverfahren nach einem der Ansprüche 1 bis 5, wobei die Stimulationsladungsrate im Bereich von mehr als 0 mC/s bis 100 mC/s liegt.

7. Kalibrierungsverfahren nach einem der Ansprüche 1 bis 6, wobei, wenn der Benutzer eine gelähmte Person oder eine Person mit einer verminderten oder fehlenden motorischen Fähigkeit oder einer verminderten oder fehlenden sensorischen Fähigkeit mindestens einer Gliedmaße ist, der Anstieg der Stimulationsladungsrate von Schritt (c) endet, wenn eine statische Kraftebene für den Muskel oder die Muskelgruppe des Körpers des Benutzers, der bzw. die die Stimulationsladungsrate empfängt, erreicht wird.

8. Kalibrierungsverfahren nach einem der Ansprüche 1 bis 6, wobei, wenn der Benutzer eine berechtigte Person ist, der Anstieg der Stimulationsladungsrate von Schritt (c) endet, wenn die Schmerzschwelle für den Muskel oder die Muskelgruppe des Körpers des Benutzers, der bzw. die die Stimulationsladungsrate empfängt, erreicht wird.

9. Computerprogramm in einem motorisierten Ergometer, vorzugsweise in einem motorisierten Fahrrad-Ergometer, das mit einem FES-System ausgestattet ist, wobei das Computerprogramm Anweisungen umfasst, die, wenn das Programm von einem Computer ausgeführt wird, den Computer dazu veranlassen, die Schritte des Kalibrierungsverfahrens nach einem der Ansprüche 1 bis 8 durchzuführen.

10. Computerprogramm nach Anspruch 9, ferner umfassend ein vom Computer ausgeführtes Programm, das den Computer dazu veranlasst, ein Verfahren zum Bestimmen der Stimulationsintervalle für eine periodische Bewegung für mindestens einen Muskel oder eine Muskelgruppe des Körpers des Benutzers als in Abhängigkeit vom Fortschrittsgrad der periodischen Bewegung auf dem Ergometer durchzuführen; wobei das Verfahren nach dem Durchführen der Schritte des Kalibrierungsverfahrens nach einem der Ansprüche 1 bis 8 durchgeführt wird.

11. Motorisiertes Ergometer zur elektrischen Stimulation, umfassend:
- einen Motor, der dazu konfiguriert ist, mindestens ein Gelenkstellglied, das mindestens einen Positionssensor umfasst, in einer periodischen Bewegung zu bewegen, wobei das Gelenkstellglied in einer Gebrauchsposition blockiert werden kann, um die Gliedmaße des Benutzers ruhigzustellen;
- ein funktionelles elektrisches Stimulationssystem (FES-System), das Folgendes umfasst:
- ein Modul, vorzugsweise ein computerbasiertes Modul, das ein Datenerfassungssystem und eine FES-Steuerung umfasst; und
- einen Stimulator, der mit zwei oder mehr elektrischen Stimulationselektroden verbunden ist; wobei die elektrischen Stimulationselektroden dazu konfiguriert sind, an mindestens einem Muskel oder einer Muskelgruppe des Körpers eines Benutzers zu haften, der bzw. die in eine periodische Bewegung durch das motorisierte Ergometer eingebunden ist, und eine Schnittstelle zum Übertragen elektrischer Impulse vom Stimulator an den Muskel oder die Muskelgruppe des Körpers des Benutzers bereitzustellen;
wobei:
die FES-Steuerung Anweisungen umfasst, um das Kalibrierungsverfahren zum Bestimmen eines Profils der Stimulationsladungsrate von mindestens einem Muskel oder einer Muskelgruppe des Körpers eines Benutzers nach einem der Ansprüche 1 bis 8 zu betreiben.

12. Motorisiertes Ergometer zur elektrischen Stimulation nach Anspruch 11, wobei das motorisierte Ergometer dazu konfiguriert ist, das Kalibrierungsverfahren nach einem der Ansprüche 1 bis 8 durchzuführen, bevor ein Benutzer ein Trainingsprogramm durchführt.

13. Motorisiertes Ergometer zur elektrischen Stimulation nach Anspruch 11 oder Anspruch 12, wobei der Muskel oder die Muskelgruppe des Körpers des Benutzers aus Folgendem ausgewählt ist: Quadrizeps (Vastus lateralis), Quadrizeps (Vastus medialis), Quadrizeps (Rectus femoris), hinteren Oberschenkelmuskulatur, Gluteus, Gastrocnemius und Tibialis anterior.

14. Motorisiertes Ergometer zur elektrischen Stimulation nach einem der Ansprüche 11 bis 13, wobei das FES-System eine Benutzerschnittstelle für Folgendes umfasst:
- Durchführen des Kalibrierungsverfahrens nach einem der Ansprüche 1 bis 8,
- gegebenenfalls, in einem zweiten Schritt, Durchführen eines Verfahrens zum Bestimmen der Stimulationsintervalle für eine periodische Bewegung für mindestens einen Muskel oder eine Muskelgruppe des Körpers des Benutzers in Abhängigkeit vom Fortschrittsgrad der periodischen Bewegung auf dem Ergometer und in Abhängigkeit von der Art des Trainingsprogramms; und
- gegebenenfalls, in einem letzten Schritt, Durchführen eines Trainingsprogramms.

15. Motorisiertes Ergometer zur elektrischen Stimulation nach Anspruch 14, wobei die Stimulationsladungsrate, die vom FES-System während des Kalibrierungsverfahrens zum Bestimmen von Stimulationsintervallen einer periodischen Bewegung für mindestens einen Muskel oder eine Muskelgruppe des Körpers des Benutzers oder während des Trainingsprogramms bereitgestellt wird, von der minimalen Stimulationsladungsrate (Cₘᵢₙ) bis zur maximalen Stimulationsladungsrate (Cₘₐₓ) reicht, die für jeden Muskel oder jede Muskelgruppe jedes Benutzers des Ergometers während des Schritts (e) des Verfahrens nach Anspruch 2 bestimmt werden.

## Revendications

1. Un procédé de calibration pour déterminer un profil de taux de charge de stimulation pour au moins un muscle ou un groupe de muscles du corps d'un utilisateur, dans un ergomètre motorisé équipé d'un système de stimulation électrique fonctionnelle (SEF); ledit ergomètre motorisé comprenant un moteur configuré pour déplacer au moins un actionneur articulé; ladite charge de stimulation étant fournie par le système SEF et étant personnalisée au corps de l'utilisateur; ledit procédé comprenant les étapes suivantes :
- Étape (a) : le placement des membres de l'utilisateur dans une position d'utilisation de l'ergomètre ;
- Étape (b) : l'immobilisation des membres de l'utilisateur dans la position d'utilisation de l'étape (a) en bloquant ledit actionneur articulé ; et
- Étape (c) : la mise en oeuvre par le système SEF d'une rampe de taux de charge de stimulation pour au moins un muscle ou un groupe de muscles des membres de l'utilisateur de l'étape (b) afin d'obtenir un profil de force statique pour ledit muscle ou groupe de muscles en fonction du taux de charge de stimulation de la rampe; ladite force statique étant la force appliquée par les membres de l'utilisateur sur une partie de l'ergomètre comprenant au moins un capteur de force; ladite étape étant mise en oeuvre lorsque les membres de l'utilisateur sont immobilisés dans la position de l'étape (b); et
- Étape (d) : éventuellement, la répétition de l'étape (c) pour chaque muscle ou groupe de muscles des membres de l'utilisateur pour lesquels une stimulation par le système SEF doit être fournie lors de l'utilisation de l'ergomètre.

2. Le procédé de calibration selon la revendication **1,** comprenant en outre une étape (e) de détermination à la fois du taux de charge de stimulation minimal (Cmin) nécessaire pour induire le début de la contraction dudit muscle ou groupe de muscles, et du taux de charge de stimulation maximal (Cmax) pour ledit muscle ou groupe de muscles correspondant à la charge nécessaire pour atteindre le seuil de douleur dudit muscle ou groupe de muscles et/ou entraînant un plafonnement de la force générée par la contraction dudit muscle ou groupe de muscles sur l'ergomètre.

3. Le procédé de calibration selon la revendication **1** ou **2,** dans lequel les membres de l'utilisateur sont les jambes de l'utilisateur, et dans lequel l'étape (c) est mise en oeuvre simultanément pour le même type de muscle ou groupe de muscles des deux jambes de l'utilisateur.

4. Le procédé de calibration selon l'une quelconque des revendications **1 à 3,** dans lequel, à l'étape (c), la rampe de taux de charge de stimulation se termine lorsqu'un plateau de force statique et/ou lorsque le seuil de douleur pour le muscle ou groupe de muscles du corps de l'utilisateur recevant le taux de charge de stimulation est atteint.

5. Le procédé de calibration selon l'une quelconque des revendications **1** à **4,** dans lequel la durée de la rampe de taux de charge de stimulation varie de 1s à 60s, de préférence est de 10s.

6. Le procédé de calibration selon l'une quelconque des revendications **1 à 5,** dans lequel le taux de charge de stimulation varie de plus de 0 mC/s à 100 mC/s.

7. Le procédé de calibration selon l'une quelconque des revendications **1** à **6,** dans lequel, lorsque l'utilisateur est une personne paralysée ou une personne ayant une capacité motrice dégradée ou absente, une capacité sensorielle dégradée ou absente d'au moins un membre, la rampe de taux de charge de stimulation de l'étape (c) se termine lorsqu'un plateau de force statique pour le muscle ou groupe de muscles du corps de l'utilisateur recevant le taux de charge de stimulation est atteint.

8. Le procédé de calibration selon l'une quelconque des revendications **1** à **6,** dans lequel, lorsque l'utilisateur est une personne valide, la rampe de taux de charge de stimulation de l'étape (c) se termine lorsque le seuil de douleur pour le muscle ou groupe de muscles du corps de l'utilisateur recevant le taux de charge de stimulation est atteint.

9. Un programme d'ordinateur dans un ergomètre motorisé, de préférence dans un ergomètre cycliste motorisé, équipé d'un système SEF, ledit programme d'ordinateur comprenant des instructions qui, lorsque le programme est exécuté par un ordinateur, amènent l'ordinateur à exécuter les étapes du procédé de calibration selon l'une quelconque des revendications **1** à **8.**

10. Le programme d'ordinateur selon la revendication **9,** comprenant en outre un programme exécuté par l'ordinateur amenant l'ordinateur à exécuter un procédé de détermination des intervalles de stimulation pour un mouvement périodique pour au moins un muscle ou un groupe de muscles du corps de l'utilisateur en fonction du degré d'avancement du mouvement périodique sur l'ergomètre; ledit procédé étant mis en oeuvre après la mise en oeuvre des étapes du procédé de calibration selon l'une quelconque des revendications **1** à **8.**

11. Un ergomètre motorisé à stimulation électrique comprenant :
- un moteur configuré pour déplacer en un mouvement périodique au moins un actionneur articulé comprenant au moins un capteur de position, ledit actionneur articulé pouvant être bloqué dans une position d'utilisation de manière à immobiliser le membre de l'utilisateur ;
- un système de stimulation électrique fonctionnelle (SEF) comprenant :
- un module, de préférence un module informatique, comprenant un système d'acquisition de données et un contrôleur SEF ; et
- un stimulateur connecté à deux électrodes de stimulation électrique ou plus ; lesdites électrodes de stimulation électrique étant configurées pour adhérer à au moins un muscle ou groupe de muscles du corps d'un utilisateur engagé dans un mouvement périodique par l'ergomètre motorisé, et pour fournir une interface permettant de transmettre des impulsions électriques du stimulateur audit muscle ou groupe de muscles du corps de l'utilisateur ;
dans lequel le contrôleur SEF comprend des instructions pour mettre en oeuvre le procédé de calibration pour déterminer un profil de taux de charge de stimulation d'au moins un muscle ou groupe de muscles du corps de l'utilisateur selon l'une quelconque des revendications **1** à **8.**

12. L'ergomètre motorisé à stimulation électrique selon la revendication **11,** dans lequel ledit ergomètre motorisé est configuré pour mettre en oeuvre le procédé selon l'une quelconque des revendications **1** à **8,** avant la mise en oeuvre d'un régime d'exercice par un utilisateur.

13. L'ergomètre motorisé à stimulation électrique selon la revendication **11** ou **12,** dans lequel le muscle ou groupe de muscles du corps de l'utilisateur est sélectionné parmi : quadriceps (vaste latéral), quadriceps (vaste médial), quadriceps (droit fémoral), ischio-jambiers, fessiers, gastrocnémien et tibial antérieur.

14. L'ergomètre motorisé à stimulation électrique selon l'une quelconque des revendications **11** à **13,** dans lequel le système SEF comprend une interface utilisateur pour :
- mettre en oeuvre le procédé de calibration selon l'une quelconque des revendications **1** à **8,**
- optionnellement, dans une deuxième étape, mettre en oeuvre un procédé de détermination des intervalles de stimulation pour un mouvement périodique pour au moins un muscle ou groupe de muscles du corps de l'utilisateur en fonction du degré d' avancement du mouvement périodique sur l'ergomètre et en fonction du type de régime d'exercice ; et
- optionnellement, dans une dernière étape, mettre en oeuvre un régime d'exercice.

15. L'ergomètre motorisé à stimulation électrique selon la revendication **14,** dans lequel le taux de charge de stimulation fourni par le système SEF pendant le procédé de détermination des intervalles de stimulation d'un mouvement périodique pour au moins un muscle ou groupe de muscles du corps de l'utilisateur ou pendant le régime d'exercice varie du taux de charge de stimulation minimal (Cmin) au taux de charge de stimulation maximal (Cmax) déterminé pour chaque muscle ou groupe de muscles de chaque utilisateur de l'ergomètre pendant l'étape (e) du procédé selon la revendication **2.**
